# EUROPEAN PATENT SPECIFICATION

(11) **EP 1 777 288 B1**
(45) Date of publication and mention of the grant of the patent: **22.07.2009**
(21) Application number: 05751043.0
(22) Date of filing: 06.06.2005
(51) Int. Cl.: C11D 10/02, C11D 1/835, C11D 1/94, C11D 3/20, C11D 3/39, A61M 1/14

(54) **CLEANSER FOR ORGANIAC/INORGANIC COMPLEX STAINS AND METHOD OF CLEANING ARTIFICIAL DIALYZER**
REINIGER FÜR KOMPLEXE ORGANISCHE/ANORGANISCHE FLECKEN UND VERFAHREN ZUM REINIGEN EINES KÜNSTLICHEN DIALYSATORS
NETTOYANT POUR TÂCHES COMPLEXES ORGANIQUES/INORGANIQUES ET PROCÉDÉ DE NETTOYAGE DE DIALYSEUR ARTIFICIEL

(30) Priority: 28.07.2004 JP 2004219668
(43) Date of publication of application: 25.04.2007
(73) Proprietor: Adeka Corporation, Tokyo 116-8554 (JP)
(72) Inventor: ZUSHI, Takehiro, Adeka Corporation, Tokyo 1168554 (JP); HUKAI, Tadahiro, Adeka Corporation, Tokyo 1168554 (JP); KAWAMATA, Hiromasa, Adeka Corporation, Tokyo 1168554 (JP)
(74) Representative: Blodig, Wolfgang
(86) International application number: PCT/JP2005/010342
(87) International publication number: WO 2006/011305

(56) References cited:
- WO-A-02/12431
- FR-A1- 2 772 620
- JP-A- 5 209 196
- JP-A- 7 233 396
- JP-A- 9 075 688
- JP-A- 11 116 990
- JP-A- 53 070 191
- JP-A- 2000 051 350
- JP-A- 2001 070 441
- JP-A- 2001 072 996
- JP-A- 2004 182 673

## Description

### Technical Field

This invention relates to cleansers for organic-inorganic composite fouling, especially those useful for the removal of organic-inorganic composite fouling occurred in tubings and the like of artificial dialyzers, and also to a method of cleaning such artificial dialyzers.

### Background Art

An artificial dialyzer is an apparatus that brings dialysis fluid and blood into contact with each other via a semipermeable membrane to effect dialysis of the blood. Such an artificial dialyzer is, however, accompanied by a problem in that, when dialysis is repeated, inorganic fouling such as calcium carbonate and calcium phosphate and organic fouling of proteins and lipids deposit in its tubing and the like. As disclosed in Patent Document 1, a cleanser has, therefore, been developed with a view to exhibiting both disinfectant activities and inorganic fouling removability by the addition of peracetic acid and hydrogen peroxide and also removing organic fouling by the addition of a nonionic surfactant.

The cleanser disclosed in Patent Document 1 is good in disinfectant activities, inorganic fouling removability, and the removability of protein fouling among organic fouling. When fouling has proceeded further andbiofilms have occurred as organic fouling, however, any attempt to remove composite fouling of the biofilms and inorganic fouling such as calcium carbonate and calcium phosphate by uing the cleanser alone results in insufficient removability of the fouling.

As a disinfectant cleanser containing peracetic acid, acetic acid, hydrogen peroxide and a surfactant (and including artificial dialyzers as an application field), Patent Document 2 discloses a composition which additionally contains a surfactant of the amine oxide type and a nonionic surfactant. This composition is, however, not described to be effective for the removal of organic-inorganic composite fouling of the above-mentioned type. Further, Patent Document 3 is a relevant application by the subject applicant.
Patent Document 1: JP-A-2001-72996
Patent Document 2: JP-A-11-116990
Patent Document 3: JP-A-2004-285154

### Disclosure of the Invention

### Object to be Achieved by the Invention

On object of the present invention is to provide a cleanser for organic-inorganic composite fouling, which has disinfectant activities and, especially when biofilms have occurred as organic fouling, can effectively remove by itself composite fouling of the biofilms and inorganic fouling such as calcium carbonate and calcium phosphate.

### Means for Achieving the Object

A cleanser according to the present invention for organic-inorganic composite fouling is an aqueous solution which contains 4 to 12 wt.% of hydrogen peroxide, 10 to 50 wt.% of acetic acid, 0.3 to 6 wt.% of peracetic acid, 4-6 wt.% of a nonionic surfactant, and 1 to 6 wt.% of a cationic surfactant.

### Advantageous Effects of the Invention

The present invention have provided as advantageous effects thereof a cleanser for organic-inorganic composite fouling, which has disinfectant activities, inorganic fouling removability and organic fouling removability and in particular, permits effective removal even when organic fouling comprises biofilms, and a method of cleaning an artificial dialyzer by using the cleanser.

### Best Modes for Carrying out the Invention

The present invention will hereinafter be described in further detail based on best modes for carrying it out. Any hydrogen peroxide, acetic acid and peracetic acid can be used in the cleanser according to the present invention for organic-inorganic composite fouling insofar as they are conventionally known.

It is suited from the standpoints of disinfectant activities and inorganic fouling removability that the cleanser according to the present invention for organic-inorganic composite fouling contains 4 to 12 wt.% (more preferably 5 to 7 wt.%) of hydrogen peroxide, 10 to 50 wt.% (more preferably 20 to 40 wt.%) of acetic acid, and 0.3 to 6 wt.% (more preferably 1 to 5 wt.%) of peracetic acid. From the standpoint of enhancing the penetrability of the cleanser into organic fouling, on the other hand, it is preferred to contain the surfactant components in a total amount of from 0.01 to 20 wt.%.

The surfactant components for use in the present invention are a nonionic surfactant and a cationic and optionally amphoteric surfactant.
As the nonionic surfactant, a nonionic surfactant which is employed in industrial applications is generally usable. It is, however, preferred to use a compound represented by the following formula (1):

R¹O-(RO)ₘ-H (1)

wherein R¹ represents a hydrocarbon group, (RO)ₘ represents a group formed by block or random polymerization of an alkylene oxide such as ethylene oxide, propylene oxide, butylene oxide, an α-olefin oxide or styrene oxide, and m stands for a number of 1 or greater, preferably 1 to 200, more preferably 2 to 100, still more preferably 5 to 15, most preferably 7 to 15.

Examples of the hydrocarbon group include alkyl groups, alkenyl groups, aryl groups, cycloalkyl groups, and cycloalkenyl groups. Illustrative alkylgroupsare methyl, ethyl, propyl, isopropyl, butyl, isobutyl, sec.-butyl, tert.-butyl, pentyl, isopentyl, sec.-pentyl, neopentyl, tert-pentyl, hexyl, sec.-hexyl, heptyl, sec.-heptyl, octyl, 2-ethylhexyl, sec.-octyl, nonyl, sec.-nonyl, decyl, sec.-decyl, undecyl, sec.-undecyl, dodecyl, sec.-dodecyl, tridecyl, isotridecyl, sec.-tridecyl, tetradecyl, sec.-tetradecyl, hexadecyl, sec.-hexadecyl, stearyl, icosyl, docosyl, tetracosyl, triacontyl, 2-butyloctyl, 2-butyldecyl, 2-hexyloctyl, 2-hexyldecyl, 2-octyldecyl, 2-hexyldodecyl, 2-octyldodeecyl, 2-decyltetradecyl, 2-dodecylhexadecyl, 2-hexadecyloctadecyl, 2-tetradecyloctadecyl, and monomethyl-branched isostearyl.

Illustrative alkenyl groups are vinyl, allyl, propenyl, butenyl, isobutenyl, pentenyl, isopentenyl, hexenyl, heptenyl, octenyl, nonenyl, decenyl, undecenyl, dodecenyl, tetradecenyl, and oleyl.

Illustrative aryl groups are phenyl, toluyl, xylyl, cumenyl, mesityl, benzyl, phenethyl, styryl, cinnamyl, benzhydryl,trityl,ethylphenyl,propylphenyl,butylphenyl, pentylphenyl, hexylphenyl, heptylphenyl, octylphenyl, nonylphenyl, decylphenyl, undecylphenyl, dodecylphenyl, phenylphenyl, benzylphenyl, styrenated phenyl, p-cumylphenyl, α-naphthyl, and β-naphthyl.

Illustrative cycloalkyl groups and cycloalkenyl groups are cyclopentyl, cyclohexyl, cycloheptyl, methylcyclopentyl, methylcyclohexyl, methylcycloheptyl, cyclopentenyl, cyclohexenyl, cycloheptenyl, methylcyclopentenyl, methylcyclohexenyl, and methylcycloheptenyl.

Among these, preferred as R¹ are alkyl, alkenyl and aryl groups, more preferred are C₆₋₂₂ alkyl, alkenyl and aryl groups, and most preferred are C₆₋₁₈ alkyl and alkenyl groups.

When R¹ is a C₆₋₁₈ alkyl or alkenyl group, R¹ is generally a residual group obtained by eliminating a hydroxyl group from a higher aliphatic alcohol. Such alcohols are industrially produced as natural alcohols derived from natural oils or fats; and as single or mixed synthetic alcohols such as Ziegler alcohols (each composed of a linear primary alcohol as a principal component) each produced through a process that polymerizes ethylene by the Ziegler process, oxo-alcohols (each composed of a linear primary alcohol as a principal component with one or more branched primary alcohols mixed therein) each produced by the oxo process that reacts carbon monoxide and hydrogen to a corresponding olefin, and secondary alcohols each produced by oxidizing a corresponding paraf f in with air and containing hydroxyl groups bonded at random to a carbon chain other than its terminals. These alcohols are all usable.

R represents an alkylene group, preferably a C₂₋₄ alkylene group, more preferably an ethylene group. The moiety (R-O)ₘ in the formula (1) can be obtained by addition-polymerizing an alkylene oxide such as ethylene oxide, propylene oxide, butylene oxide, an a-olefin oxide, or styrene oxide. When the moiety (R-O)ₘ is formed by adding an alkylene oxide or the like, R is determined by the kind of the alkylene oxide or the like to be added.

No particular limitation is imposed on the type of polymerization of the alkylene oxide or the like to be added. The type of polymerization can be the homopolymerization of a single kind of alkylene oxide, or the random copolymerization, block copolymerization or random/block copolymerization of two or more alkylene oxides. As R, an ethylene group is most preferred. When R stands for two or more groups, one of the groups may preferably be an ethylene group. A cleanser according to the present invention for organic-inorganic composite fouling, which exhibits good cleanability, can be obtained when the moiety (R-O)ₘ is a polyoxyalkylene chain containing 50 to 100 mole%, preferably 60 to 100 mole% of oxyethylene groups. The polymerization degree m is a number of 1 or greater, preferably from 1 to 200, more preferably from 2 to 100, still more preferably from 5 to 15, most preferably from 7 to 15.
In the cleanser according to the present invention for organic-inorganic composite fouling, the nonionic surfactant is used in a proportion of from 4 to 6 wt.% especially from the standpoint of the removability of biofilms.

As the cationic surfactant, a cationic surfactant which is employed in industrial applications is generally usable. It is, however, preferred to use a compound represented by the following formula (2):

R²-N⁺(CₙH₂ₙ₊₁)₃Cl⁻ (2)

wherein R² represents a hydrocarbon group, and n stands for a number of from 1 to 3, more preferably from 1 to 2.

In the cleanser according to the present invention for organic-inorganic composite fouling, the cationic surfactant is used in a proportion of from 1 to 6 wt.% from the standpoint of exhibiting cleaning effects for inorganic fouling in the form of calcium phosphate and the like or for protein fouling combined with the inorganic fouling.

As the amphoteric surfactant, an amphoteric surfactant which is employed in industrial applications is generally usable. An amphoteric surfactant of the amino acid type, betaine type, sulfobetaine type or sulfoamino acid type can be used. Preferred usable examples include dodecylbis(aminoethyl)glycine, 2-alkyl-N-carboxymethyl-N-hydroxyethylimidazolinium betaine, and alkyldimethylaminoacetic acid betaine. In the cleanser according to the present invention for organic-inorganic composite fouling, it is desired to use the amphoteric surfactant in a proportion of preferably from 1 to 6 wt.%.

Among the above-described surfactants, particularly preferred cationic surfactants are didecyldimethylammonium chloride and bis(alkanoyl(3-aminopropyl))dimethylammonium chloride, and especially preferred mixtures of cationic and amphoteric surfactants are a mixture of stearyltrimethylammonium chloride and dodecylbis(aminoethyl)glycine, a mixture of stearyltrimethylammonium chloride and 2-alkyl-N-carboxymethyl-N-hydroxyethylimidazolinium betaine, and a mixture of stearyltrimethylammonium chloride and alkyldimethylaminoacetic acid betaine.

The cleanser according to the present invention for organic-inorganic composite fouling may preferably contain, in addition to the above-described essential composition, 0.01 to 0.5 wt.% of an inclusion agent from the standpoint of preventing redeposition of fouling in an artificial dialyzer to obtain still better cleaning effects.

As such an inclusion agent, any inclusion agent can be used insofar as it is internally hydrophobic, is externally hydrophilic, is high in safety and is stable to acid. Examples include cyclic oligosaccharides represented by cyclodextrins such as α-cyclodextrin, β-cyclodextrin, γ-cyclodextrin, and their branched cyclodextrins; and crown ethers.

If desired, the cleanser according to the present invention for organic-inorganic composite fouling can also contain a chelating agent to an extent not impairing the advantageous effects of the present invention from the standpoints of the stable removability of inorganic fouling and the stability of hydrogen peroxide. As the chelating agent, a conventionally-known compound can be used as desired, and its addition in a proportion of from 0.01 to 0.5 wt.% is preferred.

The cleanser according to the present invention for organic-inorganic composite fouling is in the form of an aqueous solution which contains hydrogen peroxide, acetic acid, peracetic acid and surfactants (if desired, also a an inclusion agent and/or a chelating agent) as described above. The concentration of the aqueous solution is optional, so that the aqueous solution may be marketed as a concentrate and diluted upon use for cleaning or may be marketed at a concentration suited for use in cleaning.

The aqueous solution is not unusable even if its concentration is lower than that suited for use in cleaning. Such an aqueous solution, however, requires concentration upon its use for cleaning. It is desired to market the aqueous solution at a concentration equal to the concentration suited for use in cleaning or higher.

A detailed description will next be made about the method of cleaning an artificial dialyzer, which makes use of the cleanser according to the present invention for organic-inorganic composite fouling. The artificial dialyzer-cleaning method according to the present invention comprises passing, through the tubing of the artificial dialyzer, the above-described cleanser according to the present invention for organic-inorganic composite fouling. The cleanser for organic-inorganic composite fouling may be passed in a similar manner as conventionally-employed artificial dialyzer-cleansers.

The cleanser according to the present invention for organic-inorganic compositefouling may desirably be passed through the tubing of the artificial dialyzer after diluting it with water as needed. Specifically, concerning the concentration of the artificial dialyzer-cleanser to be passed in the artificial dialyzer-cleaning method according to the present invention, it is desired to use the artificial dialyzer-cleanser in a proportion of preferably from 0.02 to 0.5 wt.%, more preferably from 0.04 to 0.3 wt.%, in terms of the concentration of hydrogen peroxide, based on the amount of the solution to be passed. A concentration of hydrogen peroxide excessively lower than the above-described level may lead to insufficient cleanability or disinfectant activities in some instances. A concentration of hydrogen peroxide excessively higher than the above-described level, on the other hand, may result in the occurrence of bubbling in the tubing of the artificial dialyzer and/or careless deterioration in the materials of the artificial dialyzer in some instances. Accordingly, hydrogen peroxide concentrations outside the above range may not be suited in some instances.

In the artificial dialyzer-cleaning method according to the present invention, no particular limitation is imposed on the temperature of the artificial dialyzer-cleanser to be passed, and the artificial dialyzer-cleanser may be set at a similar temperature level as the conventionally-employed artificial dialyzer-cleansers. In general, the artificial dialyzer-cleanser can be conveniently used, for example, at a temperature of from 25 to 40°C.

### Example

The present invention will hereinafter be described further based on Example.

### Example

Cleansers according to the present invention for organic-inorganic composite fouling, the compositions of which contained the various components shown in Table 1-1, were prepared. After the cleansers were diluted 50-fold in water, they were promptly tested for cleanability and disinfectant activities by the methods to be described below. The results are shown in Table 1-2.

### Test for cleanability

Through 10 cm-long sections of a used silicone tube disconnected from a dialysis fluid outlet of an artificial dialyzer and carrying inorganic fouling and biofilms firmly deposited thereon, the cleaning solutions (200 mL) were circulated for 20 minutes by peristaltic pumps, respectively. The tube sections were washed with water, dried, and then visually ranked for any remaining fouling.

### Test for disinfectant activities

Purified water was sealed in the thus-washed tube sections, followed by an investigation for the growth of microorganisms. Growth or non-growth of microorganisms was visually determined based on colonies per unit area (4 cm²) after the elapse of a predetermined time.

**Table 1-1**

| | | Wt.% |
|---|---|---|
| Cleansers for organic-inorganic composite fouling | Peracetic acid | 2 |
| | Hydrogen peroxide | 5 |
| | Acetic acid | 28 |
| | Nonionic surfactant (C11 alcohol + 9EO) | 5 |
| | α-Cyclodextrin | 0.2 |
| | 1-Hydroxyethylidene-1,1-diphosphon ic acid | 0.2 |
| | Cationic and/or amphoteric surfactant (below-described Table 1-2) | 5 |
| | Water | Balance |

| | | |
|---|---|---|
| EO: ethylene oxide | | |

**Table 1-2**

| | | Cleanability | Disinfectant activities |
|---|---|---|---|
| Cationic | Stearyltrimethylammonium chloride | B | A |
| | Dodecyltrimethylammonium chloride | B | A |
| | Didecyldimethylammonium chloride | A | A |
| | Beef tallow alkyltrimethylammonium chloride | B | B |
| | Bis(alkanoyl(3-aminopropyl))dimethylammonium chloride | A | A |
| Amphoteric* | Dodecylbis(aminoethyl)glycine | A | B |
| | 2-Alkyl-N-carboxymethyl-N-hydroxyethylimidazolinium betaine | A | B |
| | Alkyldimethylaminoacetic acid betaine | A | B |
| Cationic + amphoteric (1:1) | Stearyltrimethylammonium chloride + dodecylbis(aminoethyl)glycine | A | A |
| | Stearyltrimethylammonium chloride + 2-alkyl-N-carboxymethyl-N-hydroxyethylimidazolinium betaine | A | A |
| | Stearyltrimethylammonium chloride + alkyldimethylaminoacetic acid betaine | A | A |

| | | | |
|---|---|---|---|
| * not within the scope of the claims | | | |

### Cleanability

| | |
|---|---|
| Non-inventive product: | Same as each cleanser described in Table 1 except for the omission of the cationic and/or amphoteric surfactant. |

C: Effects were equivalent to the non-inventive product (a weight reduction of the fouled tube section after the cleaning test was greater only by less than 50% than the weight reduction achieved with the non-inventive product).
B: Separation effects for composite fouling from the tube section were improved (a weight reduction of the fouled tube section after the cleaning test was greater by 50% or more than the weight reduction achieved with the non-inventive product).
A: Dissolving ability for composite fouling was enhanced (in addition to the above separation effects, separated fouling was in a dissolved form).

### Disinfectant activities

C: Effects were equivalent to the non-inventive product (growth of microorganisms was recognized in 10 days after the cleaning and the sealing of purified water).
B: Growth of microorganisms was not recognized for 10 days or longer after the cleaning and the sealing of purified water.
A: Growth of microorganisms was not recognized for 30 days or longer after the cleaning and the sealing of purified water.
The cleaning was conducted by circulating each cleaning solution of 50-fold dilution for 20 minutes through its corresponding 10-cm section of the fouled tube of the actually-operated artificial dialyzer and then washing the tube section with water. The washing solution was recovered to determine how much composite fouling had been separated and whether or not the thus-separated composite fouling was in a dissolved form.

### Industrial Applicability

The cleanser according to the present invention for organic-inorganic composite fouling has excellent disinfectant activities, inorganic fouling removability and organic fouling removability, and in particular, can remove such fouling even if organic fouling consists of biofilms.

## Claims

1. A cleanser for organic-inorganic composite fouling, which is in the form of an aqueous solution comprising
(i) 4 to 12 wt.% hydrogen peroxide,
(ii) 10 to 50 wt.% acetic acid,
(iii) 0.3 to 6 wt.% peracetic acid,
(iv) 4 to 6 wt.% of a nonionic surfactant, and
(v) 1 to 6 wt.% of a cationic surfactant.

2. A cleanser according to claim 1, further comprising 0.01 to 0.5 wt.% of an inclusion agent based on said aqueous solution.

3. A cleanser according to claim 1, further comprising 0.01 to 0.5 wt.% of a chelating agent based on said aqueous solution.

4. A cleanser according to claim 1, wherein said aqueous solution further comprises an amphoteric surfactant and a mixture of said cationic surfactant and said amphoteric surfactant is at least one mixture selected from the group consisting of a mixture of stearyltrimethylammonium chloride and dodecylbis(aminoethyl)glycine,
a mixture of stearyltrimethylammonium chloride and 2-alkyl-*N*-carboxymethyl-N-hydroxyethylimidazolinium betaine, and a mixture of stearyltrimethylammonium chloride and alkyldimethylaminoacetic acid betaine.

5. A cleanser according to claim 1, wherein said organic-inorganic composite fouling is organic-inorganic composite fouling occurred in tubing of an artificial dialyzer.

6. A cleanser according to claim 5, wherein said organic fouling comprises biofilms occurred in tubing of an artificial dialyzer.

7. A method of cleaning an artificial dialyzer, which comprises passing through tubing of an artificial dialyzer a cleanser for organic-inorganic composite fouling according to any one of claims 1-6 .

## Patentansprüche

1. Reinigungsmittel für organisch-anorganische Kompositablagerungen, das in Form einer wässrigen Lösung vorliegt und umfasst
(i) 4-12 Gew.-% Wasserstoffperoxid,
(ii) 10 -50 Gew.-% Essigsäure,
(iii) 0,3 bis 6 Gew.-% Peressigsäure,
(iv) 4-6 Gew.-% eines nicht-ionischen Tensids und
(v) 1-6 Gew.-% eines kationischen Tensids.

2. Reinigungsmittel nach Anspruch 1, das weiterhin, bezogen auf die wässrige Lösung, 0,01 bis 0,5 Gew.-% eines Inklusionsmittels umfasst.

3. Reinigungsmittel nach Anspruch 1, das weiterhin, bezogen auf die wässrige Lösung, 0,01 bis 0,5 Gew.-% eines Chelatisierungsmittel umfasst.

4. Reinigungsmittel nach Anspruch 1, wobei die wässrige Lösung weiterhin ein amphoteres Tensid umfasst, wobei ein Gemisch aus dem kationischen Tensid und dem amphoterischen Tensid mindestens ein Gemisch ist, das ausgewählt wird aus der Gruppe, bestehend aus einem Gemisch aus Stearyltrimethylammoniumchlorid und Dodecylbis(aminoethyl)glycin, einem Gemisch aus Stearyltrimethylamoniumchlorid und 2-Alkyl-N-carboxymethyl-N-hydroxyethylimidazolinium-Betain, und einem Gemisch aus Stearyltrimethylammoniumchlorid und Alkyldimethylaminoessigsäure-Betain.

5. Reinigungsmittel nach Anspruch 1, wobei die organisch-anorganische Kompositablagerung eine organisch-anorganische Kompositablagerung ist, die in Leitungen eines Dialysegeräts vorkommt.

6. Reinigungsmittel nach Anspruch 5, wobei die organischen Ablagerungen biologische Schichten umfassen, die in Leitungen eines Dialysegeräts auftreten.

7. Verfahren zum Reinigen eines Dialysegeräts, das ein Spülen eines Reinigungsmittels für organisch-anorganische Kompositablagerungen gemäß einem der Ansprüche 1 bis 6 durch Leitungen eines Dialysegeräts umfasst.

## Revendications

1. Nettoyant pour encrassement composite organique-inorganique, qui a la forme d'une solution aqueuse comprenant
(i) 4 à 12 % en poids de peroxyde d'hydrogène,
(ii) 10 à 50 % en poids d'acide acétique,
(iii) 0,3 à 6 % en poids d'acide peracétique,
(iv) 4 à 6 % en poids d'un agent tensioactif non ionique, et
(v) 1 à 6 % en poids d'un agent tensioactif cationique.

2. Nettoyant selon la revendication 1, comprenant en outre 0,01 à 0,5 % en poids d'un agent d'inclusion sur la base de ladite solution aqueuse.

3. Nettoyant selon la revendication 1, comprenant en outre 0,01 à 0,5 % en poids d'un agent chélateur sur la base de ladite solution aqueuse.

4. Nettoyant selon la revendication 1, dans lequel ladite solution aqueuse comprend en outre un agent tensioactif amphotère, et un mélange dudit agent tensioactif cationique et dudit agent tensioactif amphotère est au moins un mélange choisi dans le groupe constitué d'un mélange de chlorure de stéaryltriméthylammonium et de dodécylbis(aminoéthyl)-glycine, d'un mélange de chlorure de stéaryltriméthylammonium et de bétaïne de 2-alkyl-N-carboxyméthyl-N-hydroxyéthylimidazolinium, et d'un mélange de chlorure de stéaryltriméthylammonium et d'acide alkyldiméthylaminoacétique bétaïne.

5. Nettoyant selon la revendication 1, dans lequel ledit encrassement composite organique-inorganique est un encrassement composite organique-inorganique apparu dans la tubulure d'un dialyseur artificiel.

6. Nettoyant selon la revendication 5, dans lequel ledit encrassement organique comprend des biofilms apparus dans la tubulure d'un dialyseur artificiel.

7. Procédé pour nettoyer un dialyseur artificiel, qui comprend l'étape consistant à faire passer à travers la tubulure d'un dialyseur artificiel un nettoyant pour encrassement composite organique-inorganique selon l'une quelconque des revendications 1 à 6.
